# FASCICULE DE BREVET EUROPEEN

(11) **EP 2 121 116 B1**
(45) Date de publication et mention de la délivrance du brevet: **04.04.2012**
(21) Numéro de dépôt: 08701222.5
(22) Date de dépôt: 03.01.2008
(51) Int. Cl.: A61M 39/22

(54) **CONNECTEUR POUR ÉTABLIR UNE COMMUNICATION DE FLUIDE SOUS LE CONTRÔLE D'UNE VALVE, NOTAMMENT À USAGE DANS LE DOMAINE MÉDICAL**
VERBINDER ZUR HERSTELLUNG EINER VENTILGESTEUERTEN FLÜSSIGKEITSKOMMUNIKATION, VOR ALLEM FÜR DIE MEDIZIN
CONNECTOR FOR A ESTABLISHING A FLUID COMMUNICATION CONTROLLED BY A VALVE, ESSENTIALLY USED IN THE FIELD OF MEDICINE

(30) Priorité: 03.01.2007 FR 0700022
(43) Date de publication de la demande: 25.11.2009
(73) Titulaire: Vygon, 95440 Ecouen (FR)
(72) Inventeur: CARREZ, Jean-Luc, 95440 Ecouen (FR); DALLE, Valéry, 59170 Croix (FR); GUYOMARC'H, Pierrick, 95120 Ermont (FR); HUET, Jean-Max, 92110 Clichy (FR)
(74) Mandataire: Le Forestier, Eric
(86) Numéro de dépôt international: PCT/EP2008/050040
(87) Numéro de publication internationale: WO 2008/081027

(56) Documents cités:
- WO-A-03/018105
- US-A- 6 068 011
- US-A1- 2005 151 105
- US-B1- 6 428 520

## Description

L'invention concerne un connecteur pour établir une communication de fluide sous le contrôle d'une valve, notamment à usage dans le domaine médical.

Elle concerne plus précisément un connecteur comprenant un carter rigide et une valve élastiquement déformable, le carter déterminant une chambre allongée selon un axe cette chambre ayant une extrémité distale et une extrémité proximale, et étant accessible d'une part par un embout fixe en saillie axiale dans l'extrémité distale de la chambre et d'autre part par un conduit axial formé à l'extrémité proximale de la chambre et dans lequel peut être introduit un embout mobile, et la valve étant un corps tubulaire monobloc en matière élastomère à paroi étanche logé dans la chambre, cette valve ayant une extrémité distale constituant un espace tubulaire dans lequel pénètre ledit embout fixe et ayant une extrémité opposée conformée notamment pour constituer un bouchon dans ledit conduit axial, ce bouchon étant fendu pour communiquer avec ledit espace tubulaire, la valve et le carter étant conformés et dimensionnés en sorte que le bouchon soit élastiquement déplaçable entre une position de fermeture où il est en contact étanche avec la paroi du conduit et est contraint latéralement par cette paroi à fermer la fente et une position d'ouverture où le bouchon n'est plus contraint latéralement par la paroi du conduit en sorte que sa fente peut s'ouvrir.

Parmi les connecteurs de ce type, elle concerne ceux dont la valve est déformable élastiquement dans ladite chambre lorsqu'elle est poussée par l'embout mobile introduit dans ledit conduit en créant un effet ressort qui lui permet de reprendre sa forme initiale lorsque l'embout mobile est retiré dudit conduit.

Des connecteurs dont la valve a une capacité d'effet ressort sont décrits notamment dans les publications WO97/21463, WO 98/50106, US 6 068 011, EP 0 748 635, US 5 676 346, US 5 814 024, US 5 806 831.

De fait, l'invention vise à fournir un connecteur dont la valve soit monobloc étanche latéralement, soit élastiquement déformable à effet ressort, entoure l'embout fixe comme un doigt de gant, et réponde à plusieurs exigences :
- la fente de la valve doit s'ouvrir à la connexion de l'embout mobile, et découvrir complètement le trou interne de l'embout connecté, pour permettre un débit correct du liquide perfusé ;
- on doit réussir à pousser la valve et à bloquer l'embout mobile (luer mâle) avec un effort modéré de l'ordre de 30 Newtons (# 3kg) ;
- la valve ne doit pas créer un volume mort à l'intérieur du connecteur, qui est synonyme d'aspiration à la déconnexion ;
- elle doit résister à une forte contre-pression de liquide, possible avec les seringues et les rampes de robinets par exemple ;
- elle doit être facile à nettoyer, sans recoin ni trou à sa surface, pour ne pas « cacher » des bactéries avant connexion de l'embout mobile (luer mâle), elle doit de ce fait remonter toujours complètement après déconnexion.

On y parvient selon l'invention avec un connecteur caractérisé en ce que :
- la capacité de déformation à effet ressort de la valve concerne essentiellement le bouchon et une partie de la valve intermédiaire entre le bouchon et ledit espace tubulaire, la valve et l'embout fixe étant conformés pour que la valve soit serrée en permanence sur l'embout fixe sans formation d'un volume mort significatif dans ledit espace quelle que soit la déformation de la valve,
- l'embout fixe et la valve sont conformés pour que la valve soit maintenue sur l'embout fixe sans fléchir sous la poussée de l'embout mobile,
- la fente de la valve est constituée d'une fente proximale moulée en sorte que la fente soit ouverte lorsque le bouchon est hors dudit conduit et soit fermée lorsque le bouchon est contraint latéralement par la paroi du conduit, cette fente proximale étant suivie d'une fente distale percée apte à faire communiquer la fente moulée avec ledit espace lorsque la fente est ouverte, la fente percée étant fermée lorsque le bouchon est contraint dans le conduit, la fente percée et une partie de la fente moulée étant traversées par l'embout fixe lorsque le bouchon est hors dudit conduit sans que l'embout mobile ne pénètre dans la fente.

Dans des réalisations préférées, le connecteur présente encore une ou plusieurs des caractéristiques suivante :
- l'embout fixe présente un épaulement extérieur sur lequel la valve est en butée ;
- ledit épaulement est de forme tronconique ;
- ladite partie intermédiaire de la valve présente une collerette qui coopère avec la paroi de la chambre pour empêcher une sortie de la valve hors du connecteur à la remontée de la valve sous l'effet d'une contre pression ;
- la valve présente au niveau de ladite fente percée des talons protubérants qui rentrent dans ledit conduit lorsque la valve remonte dans le conduit sous l'effet d'une contre-pression et agissent pour comprimer la fente à cet endroit.

On décrira ci-après, un exemple non limitatif d'un connecteur conforme à l'invention, en référence aux figures du dessin joint sur lequel :

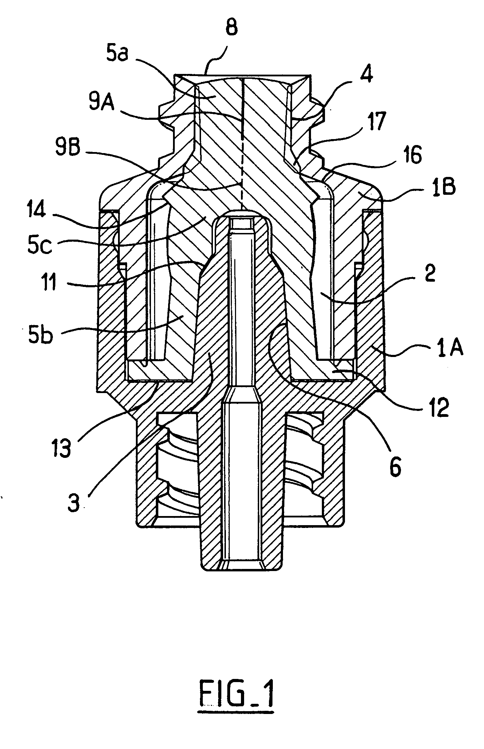

- la figure 1 est une coupe schématique axiale du connecteur au repos ;

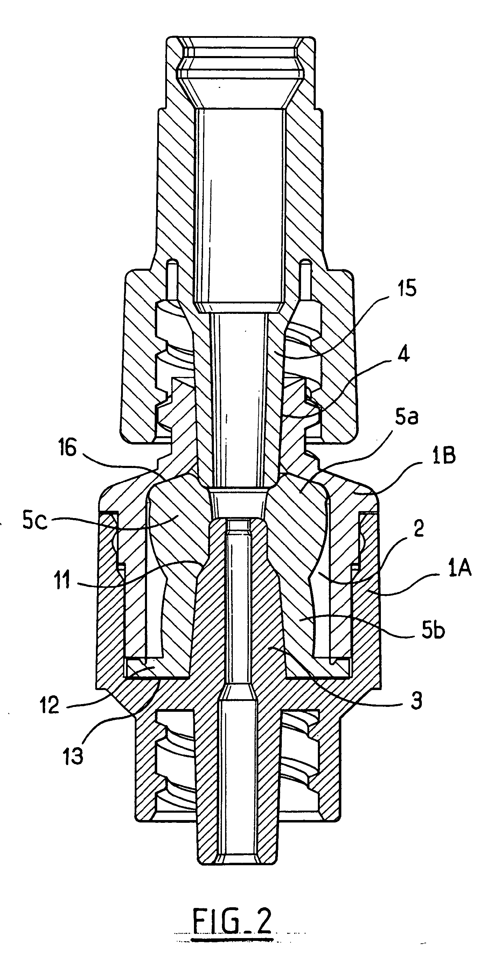

- la figure 2 est une coupe schématique axiale du connecteur en état de connexion ;
- la figure 3 est une perspective de la valve avant son montage dans le carter du connecteur ;
- la figure 4 est une coupe axiale de la valve avant son montage dans le carter du connecteur ;

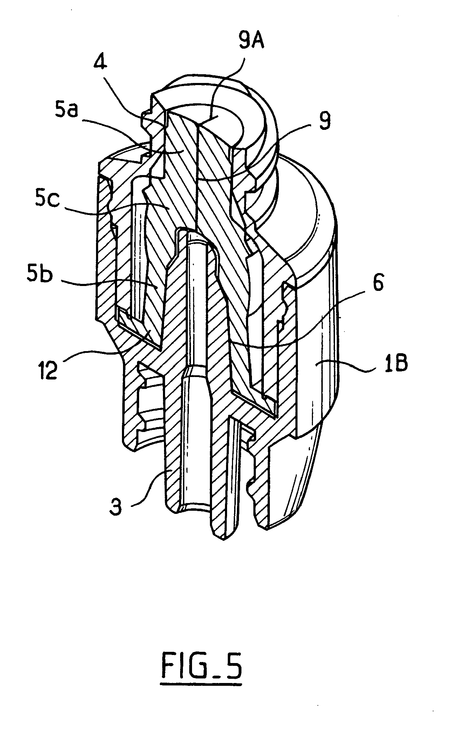

- la figure 5 est une perspective schématique coupée axialement de la valve en place et au repos dans le carter ;

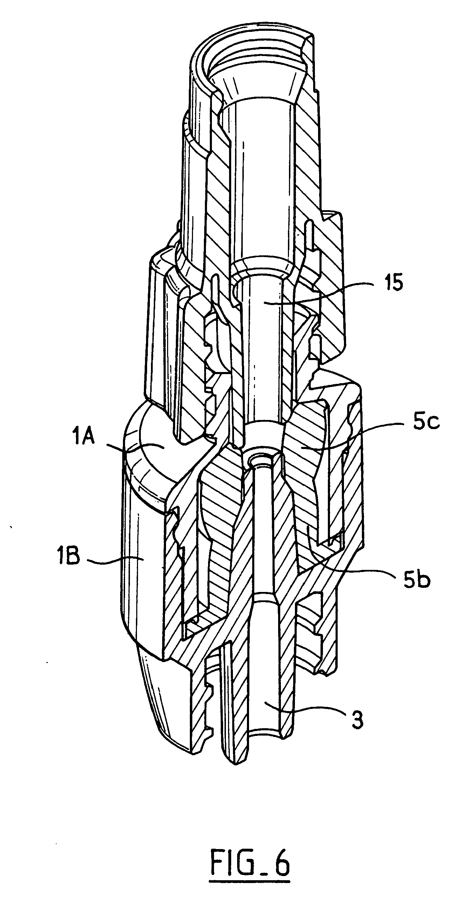

- la figure 6 est une perspective schématique coupée axialement de la valve en place dans le carter après introduction de l'embout mobile.

Le connecteur représenté sur les figures comprend :
- un carter (1) constitué d'une partie de carter femelle (1A) et d'une partie de carter mâle (1B) fixée (vissée ou clippée ou collée ou soudée) dans la partie femelle, ces deux parties coopérant pour constituer une chambre (2) allongée selon un axe, accessible à une extrémité distale par un embout axial fixe (3) solidaire de la partie de carter femelle et accessible à son extrémité opposée proximale par un conduit axial (4) formé dans la partie de carter mâle ;
- une valve (5) monobloc élastiquement déformable par exemple en silicone et à paroi étanche latéralement disposée dans ladite chambre, cette valve constituant à une extrémité distale (5b) un espace (6) en doigt de gant qui reçoit l'embout fixe (3) et à son extrémité proximale opposée un bouchon (5a) situé avec étanchéité latérale dans le conduit axial (4) et qui affleure à l'extrémité d'ouverture (8) de ce conduit.

La valve présente une fente (9) dont une partie est moulée (9A) et se trouve fermée dans le conduit axial lorsque la valve est au repos dans le carter et dont une partie suivante (9B) est fendue, par exemple au scalpel, pour faire communiquer la partie moulée avec l'espace (6).

La valve (5) présente, entre l'espace tubulaire (6) et le bouchon (7) une partie de valve intermédiaire (10).

La valve est conformée pour serrer étroitement l'embout fixe (3) quel que soit l'état de la valve et cet embout présente à proximité de l'extrémité proximale de l'espace (6) un épaulement tronconique (11) sur lequel s'appuie la valve et qui l'empêchera de glisser sur l'embout fixe sous la poussée de l'embout mobile.

A son extrémité distale, la valve présente une collerette latérale (12) appliquée contre la face de base (13) de la chambre et maintenue par la partie de carter mâle vissé dans la partie de carter femelle.

La partie intermédiaire (5c) de la valve est plus épaisse que la partie (5b) de la valve qui détermine l'espace (6) et présente, un épaulement (14) et, au dessus de cet épaulement, deux talons (17) de chaque côté de la fente coupée.

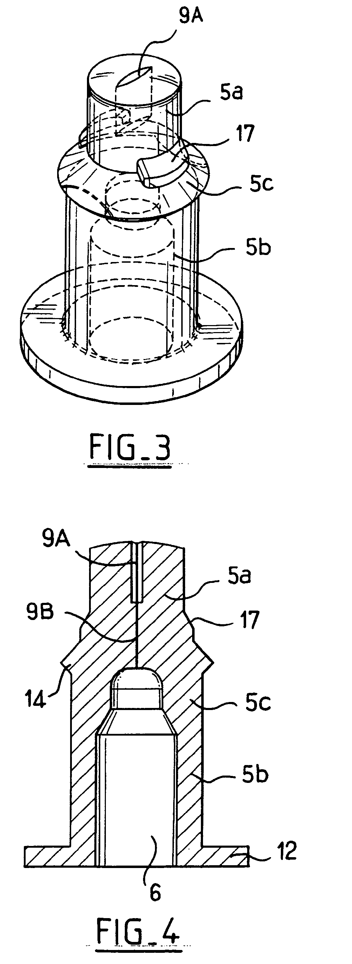

A l'état non contraint (figure 3), le bouchon (5a) a une section droite approximativement ellipsoïdale et la fente moulée (9A) a une forme approximativement ellipsoïdale, les grands axes des deux ellipses étant perpendiculaires. Lorsque la valve est contrainte dans le conduit proximal, la section ellipsoïdale du bouchon est contrainte, par son contact avec la paroi du conduit, à prendre une forme circulaire et la fente moulée (9A) est contrainte à se fermer.

Lorsque l'embout mobile (15) est introduit et poussé dans le conduit, il repousse le bouchon jusque dans la chambre, hors du conduit, en sorte que la fente moulée qui n'est plus comprimée s'ouvre d'elle-même et que la fente coupée est pénétrée par l'embout fixe, tandis que la partie intermédiaire (5c) de la valve s'expand latéralement dans la chambre en venant au contact de la paroi supérieure (16) de la chambre. En prenant appui contre la paroi du carter, la gaine emmagasine de l'énergie élastique pour assurer la fonction ressort, et ne peut pas se fermer sur elle-même grâce à la présence de l'embout fixe.

Le carter présente à la transition entre ledit conduit et ladite chambre une section droite tronconique destinée à assurer une déformation progressive du bouchon lors de l'introduction de l'embout mobile dans le conduit ou du retrait de cet embout.

A la remontée de la valve, les deux talons protubérants rentrent dans le conduit proximal du carter et sont comprimés l'un vers l'autre, ce qui comprime la fente à cet endroit et permet une bonne tenue du connecteur en contre-pression.

La collerette sur la valve fait « épaule ». Sous l'effet d'une contre-pression, la valve est poussée vers le haut (piston), l'épaule évite qu'elle s'extrude dans le conduit précédent et sorte en partie du connecteur ce qui rendrait toute reconnexion difficile.

La valve ne peut se déformer que dans sa partie intermédiaire et l'espace dans le carter le permet. Sous la poussée de l'embout mobile mâle, elle s'épanouit tout en étant écrasée sur l'épaulement de l'embout tubulaire fixe. L'étanchéité est obtenue par l'empreinte de l'embout mâle dans la valve. La course de l'embout mâle est variable en fonction de sa géométrie (tolérances mini/maxi), si le luer mâle est mini, il s'enfonce beaucoup et vient affleurer l'embout tubulaire du verrou, sinon il y a un espace entre l'embout mâle et l'embout fixe, de préférence un espace au plus égal à 1, 7 mm.

L'invention n'est pas limitée aux réalisations qui ont été décrites.

## Revendications

1. Connecteur pour établir une communication de fluide sous le contrôle d'une valve, notamment à usage dans le domaine médical, ce connecteur comprenant un carter rigide (1) et une valve (5) élastiquement déformable, le carter déterminant une chambre allongée selon un axe cette chambre ayant une extrémité distale et une extrémité proximale, et étant accessible d'une part par un embout fixe (3) en saillie axiale dans l'extrémité distale de la chambre et d'autre part par un conduit axial (4) formé à l'extrémité proximale de la chambre et dans lequel peut être introduit un embout mobile (15), et la valve (5) étant un corps tubulaire monobloc en matière élastomère à paroi étanche logé dans la chambre, cette valve ayant une extrémité distale (5b) constituant un espace tubulaire (6) dans lequel pénètre ledit embout fixe et ayant une extrémité opposée conformée notamment pour constituer un bouchon (5a) dans ledit conduit axial, ce bouchon étant fendu pour communiquer avec ledit espace tubulaire, la valve et le carter étant conformés et dimensionnés en sorte que le bouchon soit élastiquement déplaçable entre une position de fermeture où il est en contact étanche avec la paroi du conduit (4) et est contraint latéralement par cette paroi à.fermer la fente et une position d'ouverture où le bouchon n'est plus contraint latéralement par la paroi du conduit en sorte que sa fente peut s'ouvrir et cette valve étant déformable élastiquement dans ladite chambre lorsqu'elle est poussée par l'embout mobile (15) introduit dans ledit conduit en créant un effet ressort qui lui permet de reprendre sa forme initiale lorsque l'embout mobile est retiré dudit conduit, **caractérisé en ce que** :
• la capacité de déformation à effet ressort de la valve concerne essentiellement le bouchon et une partie de la valve (5c) intermédiaire entre le bouchon (5a) et ledit espace tubulaire (6), la valve et l'embout fixe (3) étant conformés pour que la valve soit serrée en permanence sur l'embout fixe sans formation d'un volume mort significatif dans ledit espace quelle que soit la déformation de la valve,
• l'embout fixe (3) et la valve (5) sont conformés pour que la valve soit maintenue sur l'embout fixe (3) sans fléchir sous la poussée de l'embout mobile,
• la fente (9) de la valve est constituée d'une fente proximale moulée (9A) en sorte que la fente soit ouverte lorsque le bouchon est hors dudit conduit et soit fermée lorsque le bouchon est contraint latéralement par la paroi du conduit, cette fente proximale étant suivie d'une fente distale (9B) percée apte à faire communiquer la fente moulée avec ledit espace lorsque la fente est ouverte, la fente percée étant fermée lorsque le bouchon est contraint dans le conduit, la fente percée et une partie de la fente moulée étant traversées par l'embout fixe (3) lorsque le bouchon est hors dudit conduit sans que l'embout mobile ne pénètre dans la fente.

2. Connecteur selon la revendication 1 dans lequel l'embout fixe (3) présente un épaulement extérieur (11) sur lequel la valve est en butée.

3. Connecteur selon la revendication 2 dans lequel ledit épaulement (11) est de forme tronconique.

4. Connecteur selon l'une des revendications 1 à 3 dont ladite partie intermédiaire (5c) de la valve présente une collerette (14) qui coopère avec la paroi de la chambre pour empêcher une sortie de la valve hors du connecteur à la remontée de la valve sous l'effet d'une contre pression.

5. Connecteur selon l'une des revendications 1 à 4 dont la valve présente au niveau de ladite fente percée des talons protubérants (17) qui rentrent dans ledit conduit lorsque la valve remonte dans le conduit sous l'effet d'une contre-pression et agissent pour comprimer la fente à cet endroit.

6. Connecteur selon l'une des revendications 1 à 5 dont le carter est constitué d'une partie de carter femelle (1A) dans laquelle est fixée une partie de carter mâle (1B), et la valve (5) présente à son extrémité distale une collerette (12) serrée autour de l'embout fixe entre ces deux parties.

7. Connecteur selon l'une des revendications 1 à 6 dont le carter présente à la transition entre ledit conduit et ladite chambre une section droite tronconique destinée à assurer une déformation progressive du bouchon (5a) lors de l'introduction ou du retrait de l'embout mobile.

8. Connecteur selon l'une des revendications 1 à 7 dont la fente moulée (9A) et le bouchon ont à l'état non contraint des sections droites approximativement ellipsoïdales, les axes des deux ellipses étant perpendiculaires.

## Claims

1. Connector for establishing a fluid communication controlled by a valve, for use in the medical field in particular, this connector comprising a rigid casing (1) and an elastically deformable valve (5), the casing defining an elongated chamber along an axis, this chamber having a distal end and a proximal end, and being accessible, on the one hand, via a fixed end piece (3) protruding axially into the distal end of the chamber and, on the other hand, by an axial channel (4) formed at the proximal end of the chamber and into which a movable end piece (15) can be inserted, and the valve (5) being a single-piece, leak-proof walled tubular body made of an elastomeric material, which is housed inside the chamber, this valve having a distal end (5b) consisting of a tubular space (6) into which said fixed end piece penetrates, and having an opposing end which is shaped in particular in order to form a plug (5a) inside said axial channel, this plug being slotted in order to communicate with said tubular space, the valve and the casing being shaped and dimensioned such that the plug is elastically movable between a closed position wherein it is in sealed contact with the wall of the channel (4) and is laterally constrained by this wall so as to close the slot, and an open position wherein the plug is no longer laterally constrained by the wall of the channel so that the slot thereof can open, and this valve being elastically deformable inside said chamber when same is pushed by the movable end piece (15) inserted into said channel while creating a spring effect which enables same to resume the initial shape thereof when the movable end piece is withdrawn from said channel, **characterised in that**:
- the spring-effect deformation capacity of the valve relates substantially to the plug and to an intermediate valve portion (5c) between the plug (5a) and said tubular space (6), the valve and the fixed end piece (3) being shaped such that the valve is continuously clamped onto the fixed end piece without forming any significant dead volume inside said space, irrespective of the deformation of the valve,
- the fixed end piece (3) and the valve (5) are shaped such that the valve is held onto the fixed end piece (3) without bending when pushed by the movable end piece,
- the valve slot (9) consists of a proximal slot (9A) moulded such that the slot is open when the plug is outside of said channel, and is closed when the plug is laterally constrained by the wall of the channel, this proximal slot being followed by a perforated distal slot (9B) capable of connecting the moulded slot with said space when the slot is open, the perforated slot, being closed when the plug is constrained inside the channel, the fixed end piece (3) passing through the perforated slot and a portion of the moulded slot when the plug is outside of said channel, without the movable plug penetrating into the slot.

2. Connector according to claim 1, wherein the fixed end piece (3) has an exterior shoulder (11) against which the valve abuts.

3. Connector according to claim 2, wherein said shoulder (11) is of a truncated cone shape.

4. Connector according to any of claims 1 to 3, the intermediate valve portion (5c) of which has a collar (14) which cooperates with the wall of the chamber in order to prevent the valve from exiting from the connector when the valve rises up again under the influence of a counter-pressure.

5. Connector according to any of claims 1 to 4, of which the valve, at the level of said perforated slot, has protruding stubs (17), which enter into said channel when the valve rises up inside the channel under the influence of a counter-pressure, and which act to compress the slot at this location.

6. Connector according to any of claims 1 to 5, the casing of which consists of a female casing portion (1A) inside which a male casing portion (1B) is fixed, and the valve (5) has a collar (12) at the distal end thereof, which is clamped around the fixed end piece between these two portions.

7. Connector according to any of claims 1 to 6, of which the casing, at the transition between said channel and said chamber, has a truncated cone-shaped straight section intended to ensure progressive deformation of the plug (5a) during insertion or withdrawal of the movable end piece.

8. Connector according to any of claims 1 to 7, the moulded slot (9A) and plug of which, in the unconstrained state, have approximately ellipsoidal straight sections, the axes of the two ellipses being perpendicular.

## Patentansprüche

1. Verbindungselement zum Herstellen eines ventilgesteuerten Fluidaustauschs, insbesondere zur Verwendung im Bereich der Medizin, wobei das Verbindungselement ein starres Gehäuse (1) und ein elastisch verformbares Ventil (5) umfasst und das Gehäuse eine in Richtung einer Achse langestreckte Kammer definiert, wobei die Kammer ein distales und ein proximales Ende hat und zum einen über ein feststehendes Mundstück (3), das am distalen Ende der Kammer axial hervorsteht, und zum anderen über einen axialen Kanal (4) zugänglich ist, der am proximalen Ende der Kammer ausgebildet ist und in den ein bewegliches Mundstück (15) eingeführt werden kann, wobei das Ventil (5) ein röhrenförmiges Element aus einem Stück und aus einem Elastomermaterial mit undurchlässigen Wänden ist, das sich in der Kammer befindet, wobei das Ventil ein distales Ende (5b) hat, das einen röhrenförmigen Raum (6) bildet, in den das feststehende Mundstück eindringt, und ein entgegengesetztes Ende hat, das insbesondere so ausgeführt ist, dass es einen Stopfen (5a) im axialen Kanal bildet, wobei der Stopfen gespalten ist, um eine Verbindung mit dem röhrenförmigen Raum herzustellen, und wobei das Ventil und das Gehäuse derart ausgeführt und bemessen sind, dass der Stopfen elastisch verschiebbar ist, und dies zwischen einer schließenden Position, in der er in abdichtendem Kontakt mit der Wand des Kanals (4) ist und in der er durch diese Wand seitlich zusammengedrückt wird, um den Spalt zu schließen, und einer öffnenden Position, in der der Stopfen durch die Wand des Kanals nicht mehr seitlich zusammengedrückt wird, derart, dass sein Spalt sich öffnen kann, und wobei das Ventil in der Kammer elastisch verformbar ist, wenn es durch das bewegliche Mundstück (15), das in den Kanal eingeführt wird, gedrückt wird, wobei eine rückfedernde Wirkung erzeugt wird, die es ihm gestattet, seine anfängliche Form wieder einzunehmen, wenn das bewegliche Mundstück aus dem Kanal herausgezogen wird, **dadurch gekennzeichnet, dass**:
- die Verformungsfähigkeit mit Rückfederwirkung des Ventils im Wesentlichen den Stopfen und einen Teil des Ventils (5c), der zwischen dem Stopfen (5a) und dem röhrenförmigen Raum (6) liegt, betrifft, wobei das Ventil und das feststehende Mundstück (3) dafür eingerichtet sind, dass das Ventil ständig eng anliegend auf dem feststehenden Mundstück sitzt, ohne dass dabei in dem Raum ein wesentliches totes Volumen entsteht, und dies unabhängig von der Verformung des Ventils,
- das feststehende Mundstück (3) und das Ventil (5) dafür eingerichtet sind, dass das Ventil auf dem feststehenden Mundstück (3) gehalten wird, und dies ohne sich unter dem Druck des beweglichen Mundstücks zu biegen,
- der Spalt (9) des Ventils durch einen geformten proximalen Spalt (9A) gebildet wird, und dies derart, dass der Spalt offen ist, wenn der Stopfen sich außerhalb des Kanals befindet, und geschlossen ist, wenn der Stopfen durch die Wand des Kanals seitlich zusammengedrückt wird, wobei auf den proximalen Spalt ein perforierter distaler Spalt (9B) folgt, der dafür eingerichtet ist, den geformten Spalt mit dem Raum zu verbinden, wenn der Spalt offen ist, wobei der perforierte Spalt geschlossen ist, wenn der Stopfen im Kanal zusammengedrückt ist, und wobei das feststehende Mundstück (3) durch den perforierte Spalt und einen Teil des geformten Spalts verläuft, wenn der Stopfen außerhalb des Kanals ist, ohne dass das bewegliche Mundstück in den Spalt eindringt.

2. Verbindungselement nach Anspruch 1, bei dem das feststehende Mundstück (3) eine äußere Schulter (11) hat, auf der das Ventil auf Anschlag sitzt.

3. Verbindungselement nach Anspruch 2, bei dem die Schulter (11) kegelstumpfförmig ist.

4. Verbindungselement nach einem der Ansprüche 1 bis 3, bei dem der zwischengeschaltete Teil (5c) des Ventils einen Kranz (14) hat, der mit der Wand der Kammer zusammenarbeitet, um beim Anheben des Ventils unter der Wirkung eines Gegendrucks ein Austreten des Ventils aus dem Verbindungselement zu verhindern.

5. Verbindungselement nach einem der Ansprüche 1 bis 4, bei dem das Ventil im Bereich des perforierten Spalts hervorstehende Absätze (17) hat, die in den Kanal eindringen, wenn das Ventil unter der Wirkung eines Gegendrucks sich den Kanal hinauf bewegt, und die bewirken, dass der Spalt an dieser Stelle zusammengedrückt wird.

6. Verbindungselement nach einem der Ansprüche 1 bis 5, bei dem das Gehäuse aus einem weiblichen Gehäuseteil (1A) besteht, in dem ein männlicher Gehäuseteil (1B) befestigt ist, wobei das Ventil (5) an seinem distalen Ende einen Kranz (12) umfasst, der um das feststehende Mundstück herum zwischen diesen beiden Teilen eingezwängt ist.

7. Verbindungselement nach einem der Ansprüche 1 bis 6, bei dem das Gehäuse am Übergang zwischen dem Kanal und der Kammer einen kegelstumpfförmigen Querschnitt hat, der dafür vorgesehen ist, eine zunehmende Verformung des Stopfens (5a) beim Einführen oder Herausziehen des beweglichen Mundstücks sicherzustellen.

8. Verbindungselement nach einem der Ansprüche 1 bis 7, bei dem der geformte Spalt (9A) und der Stopfen im nicht zusammengedrückten Zustand in etwa ellipsenförmige Querschnitte haben, wobei die Achsen der beiden Ellipsen senkrecht zueinander stehen.
